Europäisches Patentamt

European Patent Office

Office européen des brevets

(11) Publication number: **0 212 522**
**A2**

# (12) EUROPEAN PATENT APPLICATION

(21) Application number: 86111064.1

(22) Date of filing: 11.08.86

(51) Int. Cl.⁴: **G 01 N 33/74**
**// G01N33/577, G01N33/543**

(30) Priority: 12.08.85 JP 177813/85

(43) Date of publication of application: 04.03.87
**Bulletin 87/10**

(84) Designated Contracting States: **AT BE CH DE FR GB IT LI
NL SE**

(71) Applicant: **Juridical Foundation The
Chemo-Sero-Therapeutic Research Institute, 668,
Ohkubo Shimizu-machi, Kumamoto-shi Kumamoto-ken
(JP)**

(72) Inventor: **Nakashima, Toshihiro, No. 733-32,
Izumi 7-chome, Kumamoto-shi Kumamoto-ken (JP)**
Inventor: **Unoki, Masanori, No. 622-6, Shinchi
Shimizu-machi, Kumamoto-shi Kumamoto-ken (JP)**
Inventor: **Eda, Yasuyuki, No. 668, Okubo Shimizu-machi,
Kumamoto-shi Kumamoto-ken (JP)**
Inventor: **Fujita, Haruo, No. 1446-32, Kannabe-machi,
Kumamoto-shi Kumamoto-ken (JP)**

(74) Representative: **Vossius & Partner,
Siebertstrasse 4 P.O. Box 86 07 67,
D-8000 München 86 (DE)**

(54) **Method for the measurement of TSH.**

(57) Described is an improved method for the measurement of
TSH in blood or body liquid by immunoassays such as EIA and
RIA, characterized in that TSH-β subunit specific monoclonal
antibodies recognizing different epitopes are used. According
to this method, TSH can easily be measured in high sensitivity
with high specificity within a very short period of time without
inhibition by the presence of other glycoprotein hormones
such as LH, HCG and FSH. This method is useful for diagnosis
of various diseases in which TSH plays a role, such as proto-
phathic hypothyroidism, Basedow's disease and cretinism.

0212522

VOSSIUS & PARTNER
PATENTANWÄLTE
SIEBERTSTR. 4
8000 MÜNCHEN 86

u.Z: U 795 EP
Case:613391
Juridical Foundation The
Chemo-...

11. August 1986

## METHOD FOR THE MEASUREMENT OF TSH

This invention relates to a method for the measurement of hormones, more particularly a method for the measurement of TSH (thyroid stimulating hormone) by an immunoassay using monoclonal antibodies.

### Technical Background and Prior Art

It is well known that the measurement of the concentration of TSH in blood or body liquid is useful for diagnosis of protopathic hypothyroidism, Basedow's disease, cretinism, etc. Immunoassays such as EIA (enzyme immunoassay) and RIA (radioimmunoassay) are used for such a purpose.

TSH is a glycoprotein hormone., as are LH (luteinizing hormone), HCG (human chorionic gonadotropin) and FSH (follicle-stimulating hormone). These glycoprotein hormones are composed of an $\alpha$ subunit and a $\beta$ subunit. The $\alpha$ subunits of these glycoprotein hormones have very similar structure, and hence, the antigenicity of the $\alpha$ subunit of TSH is similar to that of LH, HCG and FSH. On the other hand, the $\beta$ subunits of these glycoprotein hormones have structures different from each other. When TSH in blood or body liquid, which usually contain not only TSH but also other glycoprotein hormones, is measured by the conventional EIA and RIA using polyclonal antibody, problems concerning the specificity and reproducibility may arise.

Because of the similar structure of the $\alpha$-subunits of said glycoprotein hormones, cross reaction may occur when using polyclonal antibody. From this viewpoint, the use of a TSH specific monoclonal antibody for the immunoassay has been proposed. For instance, H. Garrett, Wada et al proposed an EIA using an $\alpha$ subunit specific monoclonal antibody and a $\beta$ subunit specific monoclonal antibody of the hormones (cf. Clinical Chemistry, Vol. 28, No. 9, pp 1862 - 1866, 1982).

The $\alpha$ subunit specific monoclonal antibody is used as a solid phase and the $\beta$ subunit specific monoclonal antibody as a labelled antibody.

It has been found by the present inventors that the $\alpha$ and $\beta$ subunit specific monoclonal antibodies do not necessarily show uniform reactivity but show variant reactivities depending on the established clones.

The sandwich method of the conventional RIA or EIA, is usually carried out by the so-called "two step method" which comprises entering a test sample into a test tube, adding and reacting a solid phase adsorbent, washing the solid phase, adding and reacting a labelled antibody (or vice versa), washing the reaction product, and then measuring the amount of the labelled antibody bound to the solid phase adsorbent, or by the so-called "one step method" (also called "two site immunoassay") which comprises adding simultaneously a test sample, a solid phase adsorbent and a labelled antibody, reacting them, washing the reaction product, and then measuring the amount of the labelled antibody bound to the solid phase adsorbent. In the former method, the reaction and washing steps must be done twice and hence the operation is very troublesome and requires highly skilled technique. The latter method is simple and convenient because the reaction and washing steps are required only once, but this method is inferior in sensitivity and hence is less reliable. Thus, for the/development of a high sensitive one step method, it is very important to find an improved solid phase adsorbent and an improved labelled antibody which both have high specificity.

Under the circumstances, the present inventors had studied the measurement of TSH by an immunoassay using a monoclonal antibody and had found an improved immunoassay applicable to the one step method by using a combination of an α subunit specific monoclonal antibody and a β subunit specific monoclonal antibody having a high specificity (cf. Japanese Patent First Publication No. 57857/1986). According to this method, TSH can be measured with high sensitivity, i.e. even in a level of about 0.1 μU/ml. However, this method has still a drawback in that in view of the similar antigenicity of the α subunit to that of other glycoprotein hormones as mentioned hereinbefore, the inhibition by IH, HCG and FSH, present in the test sample can not be completely avoided.

### Object of the Invention

The present inventors assumed that one of the reasons of the variant reactivity depending on the established clones may be due to /a difference in the epitope to be recognized, and that when two β subunit specific monoclonal antibodies recognize different epitopes, a combination of these β subunit specific monoclonal antibodies may be used for EIA or RIA by a sandwich method. Based on the assumption, the present inventors have further studied and found that the desired measurement of TSH can effectively be carried out by using β subunit specific monoclonal antibodies as the solid phase antibody and as the labelled antibody, both β subunit specific monoclonal antibodies having epitopes different from each other.

An object of the present invention is to provide an improved method for the measurement of TSH in blood or body liquid without inhibition by the other glycoprotein hormones present. It is another object of the invention to provide an improved immunoassay of TSH by /a sandwich method using β subunit specific monoclonal antibodies recognizing different epitopes as the solid phase antibody and as the labelled antibody, respectively. These and other objects and advantages of the invention will be apparent to skilled persons from the following description.

## Brief Description of Drawing

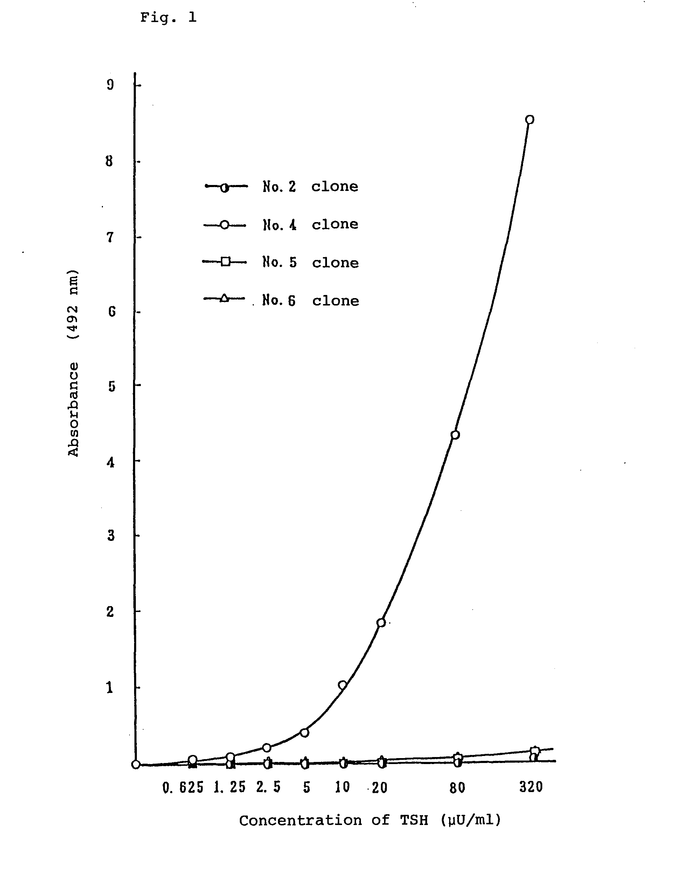

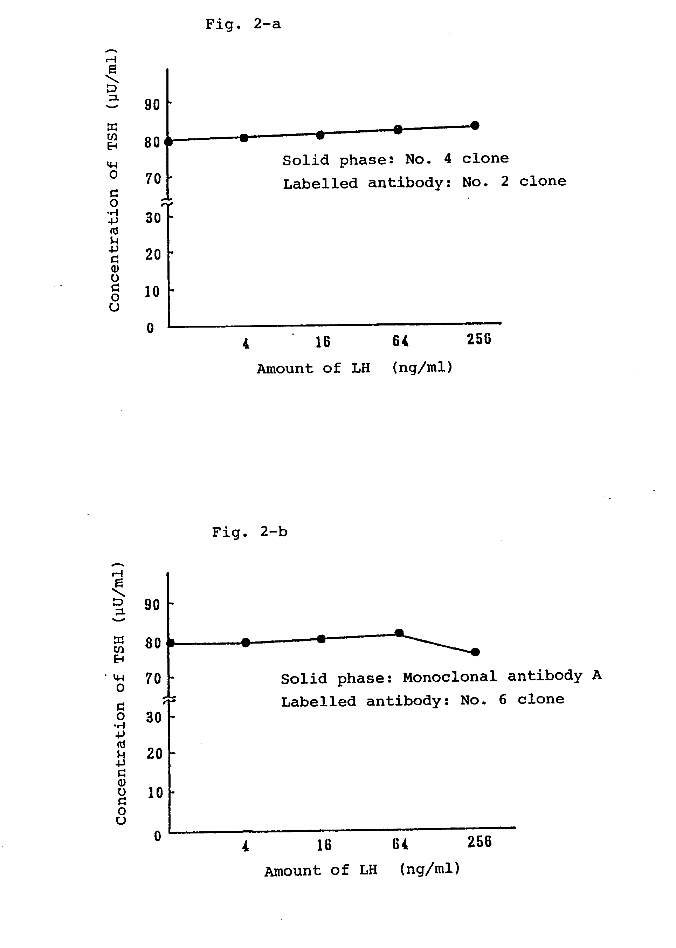

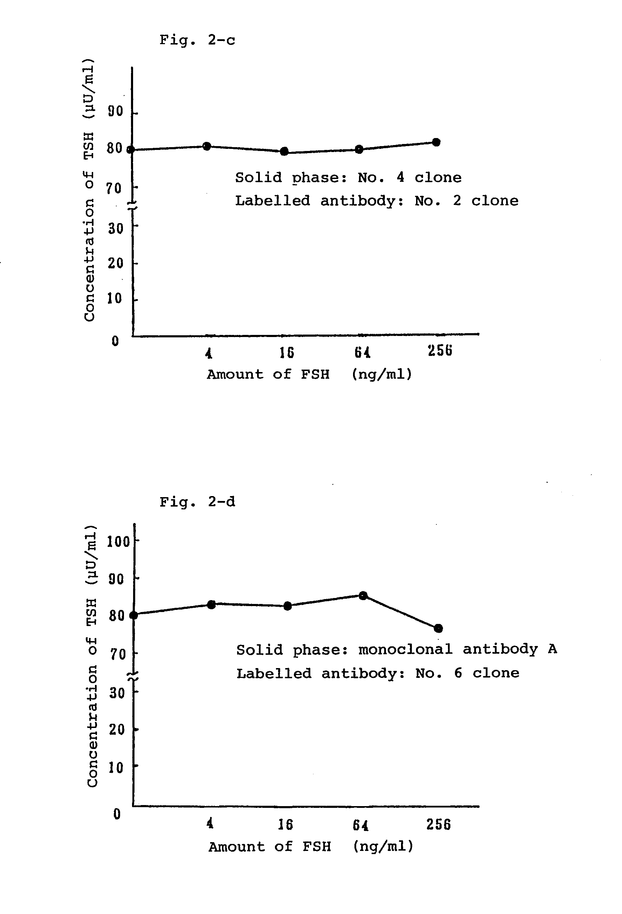

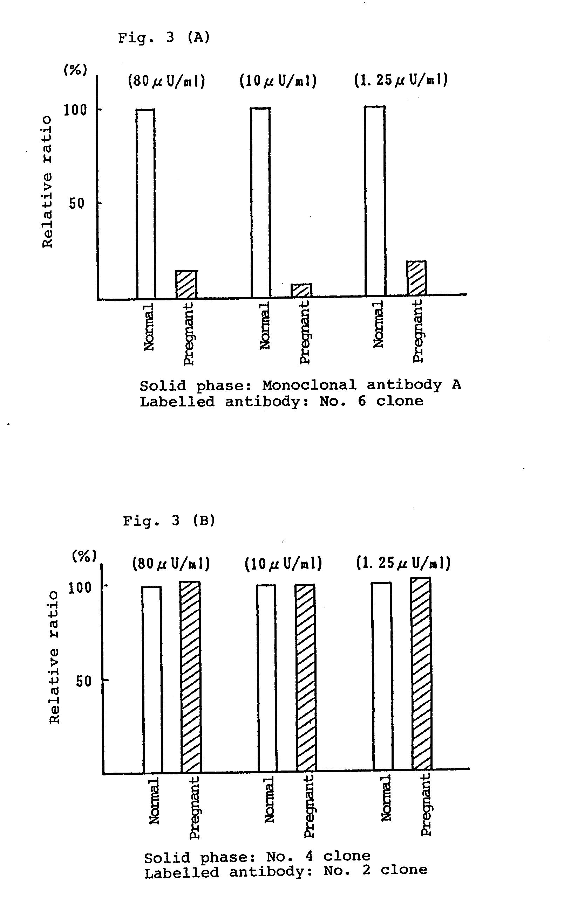

Fig. 1 shows the correlation between the absorbance of a reaction mixture and the concentration of TSH in the measurement of TSH by a one step EIA using various combinations of various TSH-$\beta$ subunit specific monoclonal antibodies. Fig. 2 shows the correlation between the concentration of TSH and the amount of the other glycoprotein hormones (LH and FSH) present, in the measurement of TSH by a one step EIA.. Fig. 2-a and Fig. 2-c illustrate the method of the present invention and Fig. 2-b and Fig. 2-d illustrate a reference method. Fig. 3 shows how the other glycoprotein hormone (HCG) present, affects the measurement of TSH in a serum containing HCG collected from a pregant woman by a one step EIA of the present invention and a reference method.

## Detailed Description of the Invention

The measurement of TSH by the present invention is carried out by the sandwich method, that is, by subjecting a test sample containing TSH (antigen) to an antigen-antibody reaction with an insolubilized antibody (the solid phase) and an enzyme- or radioisotope-labelled antibody, separating the labelled antibody bound to the antigen to be determined from the free labelled antibody, and measuring the amount of the bound antigen, which is characterized in that an insolubilized TSH-β subunit
specific monoclonal antibody is used as the solid phase and
a labelled TSH-β subunit specific monoclonal antibody is
used as the labelled antibody, said TSH-β subunit specific
monoclonal antibodies recognizing  different epitopes.


The method of this invention is preferably carried
out by/the one-step sandwich method, but can also be carried out
by/the two-step sandwich method, both in EIA and RIA.

The TSH-β subunit specific monoclonal antibodies
used in this invention can be prepared by a conventional
cell fusion method, for instance, by immunizing an
appropriate animal (e.g. mouse) with TSH or TSH-β subunit,
collecting spleen cells of the immunized animal, subjecting
the spleen cells to cell fusion with myeloma cells of an
appropriate animal (e.g. mouse), cloning the fused cells to
produce hybridoma being capable of producing monoclonal
antibody, selecting therefrom hybridoma being capable of
producing β subunit specific monoclonal antibody, followed
by growing the hybridoma in an artificial medium or in mouse
abdomen to produce the β subunit specific monoclonal
antibodies.
Two kinds of TSH-β subunit specific monoclonal antibodies
recognizing different epitopes are selected from the monoclonal antibodies obtained above. The selection can be
carried out, for instance, as follows.

One monoclonal antibody is treated with $^{125}I$-labelled TSH (as a tracer), and is then reacted with another monoclonal antibody which has previously been immobilized onto polystyrene beads. After washing the resulting solid phase, the radioactivity (cpm) of the solid phase is measured. When the radioactivity of the solid phase is high, this means that the antibodies do not compete in the antigen-antibody reaction, and hence, both antibodies recognize epitopes different from each other and are useful in this invention. On the other hand, when the radioactivity of the solid phase is low, this means that both antibodies compete in the antigen-antibody reaction and recognize similar epitope, and hence, such a combination can not be used in this invention.

One of the TSH-β subunit specific monoclonal antibodies recognizing different epitopes is insolubilized in order to use/it as a solid phase, and another one is labelled by an enzyme or a radioisotipe in order to use/it as a labelled antibody.

The insolubilization or fixation of the monoclonal antibody is done by conventional methods, for instance, by immobilizing the antibody onto a suitable fixing material , such as glass beads, silicone rod, or natural or synthetic high molecular compounds (e.g. polystyrene beads, polystyrene tube, etc.).

The labelling of the monoclonal antibody is also done by coventional methods. In the case of EIA, the antibody is labelled with an enzyme, such as alkali phosphatase, β-glucosidase, glucose oxidase, or peroxidase. In case of RIA, the antibody is labelled with a radioisotope, such as $^{125}I, ^{131}I$, using the Hunter-Greenwood method [cf. Nature, $\underline{194}$, 495 (1962)] or the Chloramine-T method [cf. F. C. Greenwood et al, Biochem. J., $\underline{89}$, 114 (1963)].

The measuring procedure of TSH by the present invention is explained below in the case of the one-step sandwich method.

A test sample containing TSH to be determined (e.g. blood or body liquid), an insolubilized TSH-β subunit specific monoclonal antibody and a labelled TSH-β subunit specific monoclonal antibody are entered into a test tube, and the mixture is incubated at an appropriate temperature (e.g. 37°C) for a few or several hours to effect the antigen-antibody reaction. After washing the resulting solid phase with a buffer (e.g. phosphate buffered saline solution, borate buffer, etc.), the amount of TSH bound to the solid phase is measured by conventional methods. In case of EIA, the enzymatic activity of the labelled antibody bound to the solid phase is measured. For instance, when a peroxidase is used for labelling of the antibody, the solid phase is reacted with $H_2O_2$ to effect a coloring reaction, and then the color is measured by colorimetry. In case of RIA, the radioactivity of the labelled antibody bound to the solid phase is counted by a conventional method.

Based on the data thus obtained, the actual amount of TSH contained in the test sample is calculated by using a calibration curve which was previously prepared with standard TSH.

According to the present invention, TSH in blood or body liquid can easily be measured in high sensitivity with high specificity within a very short period of time in immunoassays such as EIA and RIA, and hence, the method of the present invention is useful for the diagnosis of various diseases in which TSH participates, such as protopathic hypothyroidism, Bassedow's disease, and cretinism.

The present invention is illustrated by the following Preparation and Example, but should not be construed to be limited thereto.

Preparation

(1) Preparation of TSH-β subunit specific monoclonal antibodies:

TSH or TSH-β subunit (50 µg) is dissolved in 0.9 % physiological saline solution (1.3 ml), and thereto is added Freund's complete adjuvant (FCA, manufactured by DIFCO) (1.3 ml) to prepare a water-in-oil type emulsion, which is used in basic immunization.
Separately, TSH or TSH-β subunit (50 µg) is dissolved in 0.9 % physiological saline solution (1.3 ml). This solution is used in booster immunization.

BALB/c male mice (4 - 5 week age) were immunized by using the above-prepared antigen for immunizing and boostering in the manner and schedule as shown in Table 1. The spleen cells were collected from the immunized mice , and fused with commercially available mouse myeloma cells (P3-X63-Ag8-U1), followed by cloning to obtain hybridoma being capable of producing TSH-β subunit specific monoclonal antibodies, in the same manner as described in "Hybridoma and Monoclonal Antibody", ed. T. Watanabe, issued by R & D Planning Co., 1982, pages 20 - 29. The thus-obtained hybridoma being capable of producing TSH-β subunit specific monoclonal antibody were grown in mouse abdomen to produce monoclonal antibodies.

Table 1 (Immunization manner and schedule)

| Clone No. | Antigen for immu-nization | Basic immunization | | Booster immunization | | | Time of cell fusion (after booster immunization) |
|---|---|---|---|---|---|---|---|
| | | Amount of antigen | Region | Time (from basic immunization) | Amount of booster | Region | |
| 1 | TSH-β | 5 µg + FCA | Abdomen | 24th day | 5 µg | Tail vein | 4th day |
| 2 | TSH | " | " | 53rd day | 10 µg | " | " |
| 3 | TSH | " | " | 48th day | 5 µg | " | " |
| 4 | TSH | " | " | " | " | " | " |
| 5 | TSH | " | " | 60th day | 10 µg | " | " |
| 6 | TSH | " | " | " | " | " | " |

- 12 -

The specificity of the above six monoclonal antibodies was measured by RIA using as a tracer $^{125}$I-labelled TSH, $^{125}$I-labelled TSH-α and $^{125}$I-labelled TSH-β. The immunoglobulin subclass of the monoclonal antibodies was determined by the Ouchterlony method in culture supernatant of each clone using antisera against α, γ1, γ2a, γ2b, γ3, μ, κ, and λ chains.

The results shown in Table 2 make clear that the TSH-β sub-unit specific monoclonal antibodies obtained have various reactivities.

Table 2

| Clone No. | Specificity of clone | | | | Subclass of immunoglobulin | |
|-----------|-----------|--------|--------|-----------------|-----------|-----------|
| | Whole TSH | TSH-α | TSH-β | Evalu-ation | H chain | L chain |
| 1 | 4.1 | 1.4 | 13.7 | β | γ1 | κ |
| 2 | 7.0 | 1.4 | 10.8 | β | γ1 | κ |
| 3 | 17.6 | 1.3 | 10.6 | β | γ1 | κ |
| 4 | 7.0 | 1.6 | 9.6 | β | γ1 | κ |
| 5 | 10.3 | 2.2 | 26.4 | β | γ1 | κ |
| 6 | 11.1 | 2.2 | 24.6 | β | γ1 | κ |

[Note]: The value of specificity of clone is shown by a ratio of radioactivity (cpm) to the data of negative control where the culture supernatant of mouse myeloma cells (P3-X63-Ag8-U1) was used.

(2) Determination of epitopes to be recognized:

As to the six TSH-β subunit monoclonal antibodies

obtained above, the difference of epitope was determined in the following manner.

To $^{125}$I-labelled TSH (32,000 cpm, 200 μl) (as a tracer) was added one monoclonal antibody (concentration: 50 μg/ml, 200 μl), and the mixture was reacted at 37°C for 2 hours. To the reaction mixture was added another monoclonal antibody which was immobilized onto polystyrene beads, and the mixture was reacted at 37°C for 2 hours. After the solid phase was washed with PBS, the radioactivity (cpm) of the solid phase was counted. Among the various combinations of the monoclonal antibodies, one combination of the monoclonal antibodies shows high radioactivity, i.e. less competition in the reaction, i.e. the antibodies recognize different epitopes.

The results are shown in Table 3. As is clear from the results, the combination of No. 2 clone and No. 4 clone recognizes different epitopes, and other combinations recognize similar or the same epitopes.

Table 3

| Immobilized clone No. | Clone No. to be reacted | | | | | | |
|---|---|---|---|---|---|---|---|
| | Control | 1 | 2 | 3 | 4 | 5 | 6 |
| 2 | 7500 | 490 | 520 | 550 | 6500 | 890 | 520 |
| 4 | 7000 | 460 | 6950 | 720 | 460 | 1200 | 820 |

[Note]: In the control, there was used TSH-α subunit monoclonal antibody used in Example below.

## Example

The sensitivity of the combination of the two TSH-ß subunit monoclonal antibodies recognizing different epitopes (clone Nos. 2 and 4) in EIA was tested in comparison with the combinations of other two clones as follows.

Firstly, a test tube was charged with a standard TSH having a predetermined concentration (200 µl), horse radish peroxidase-labelled monoclonal antibody of No. 2 having a predetermined concentration (200 µl), and each monoclonal antibody of Nos. 2, 4, 5 and 6 which were immobilized onto polystyrene beads. The mixture was reacted at 37°C for 2 hours. The resulting polystyrene beads were washed with 5 % Tween 20-phosphate buffered saline solution (PBS) three times, and then transferred to other test tube.

To the test tube was added a mixture (500 µl) which was prepared from a solution (pH 4.8, 25 ml) containing 0.1 M citric acid and 0.2 M sodium phosphate, 5 % $H_2O_2$ (25 µl), and o-phenylenediamine dihydrochloride (15 mg). The resulting mixture was reacted in a dark room at 37°C for one hour, and thereto was added 6N $H_2SO_4$ (125 µl) in order to stop the reaction. The absorbance of the reaction mixture was measured at an optical density (O.D.) of 492 nm, and the data were plotted in a graph in correlation with the concentration of TSH. The result is shown in Fig. 1.

As is clear from Fig. 1, only the combination of clone No. 2 and clone No. 4 is useful for the measurement, but other combinations can not be used for the determination.

Besides, the sensitivity (limit for determination) in the combination of clone No. 2 and clone No. 4 was determined based on a standard curve. As a result, assuming that the limit for determination is at the absorbance of 2.1 time as much as the absorbance of the blank control, the limit for determination was 0.3 µU/ml, which means that the sensitivity is very high.

Moreover, the effect of the presence of other glycoprotein hormones such as LH, FSH and HCG was studied in case of EIA, using labelled No. 2 clone (as labelled antibody) and immobilized No. 4 clone (as solid phase) in comparison with the following system:

Labelled antibody: α-subunit specific monoclonal antibody A

Solid phase: No. 6 clone

The monoclonal antibody A had the following specificity when evaluated in the same manner as described in the above Preparation-(1), which had such a high sensitivity (limit for determination) as 0.125 µU/ml.

Sensitivity of monoclonal antibody A:

| TSH | TSH-α | TSH-β | Evaluation |
|-----|-------|-------|------------|
| 6.5 | 33.8 | 1.0 | α |

In the same manner as described above, an EIA was carried out in the above two systems wherein TSH was used in a concentration of 80 µU/ml, and other glycoprotein hormones were used in concentrations of 4, 16, 64 and 256 ng/ml.

The results are shown in Fig. 2, wherein Fig. 2-a and 2-b illustrate LH present in the system. Fig. 2-a is an embodiment of this invention using No. 4 clone as the solid phase and No. 2 clone as the labelled antibody. Fig. 2-b is an embodiment of a reference using monoclonal antibody A as the solid phase and No. 6 clone as the labelled antibody; Further, Fig. 2-c and Fig. 2-d illustrate FSH present in the system. Fig. 2-c is another embodiment : of this invention using No. 4 clone as the solid phase and No. 2 clone as the labelled antibody. Fig. 2-d is an embodiment of a reference using monoclonal antibody A as the solid phase and No. 6 clone as the labelled antibody.

As is clear from the results shown in Fig. 2, in the reference systems using monoclonal antibody A, the reaction was inhibited by the presence of other glycoprotein hormones in an amount of 64 ng/ml or more, but in the systems of this invention, no effect was observed in the presence of other glycoprotein hormones.

In a further assay, serum containing 20 mIU/ml of HCG was collected from a pregnant woman and the effect of HCG was tested likewise, whereby TSH was used in a concentration of 80, 10, or 1.25 µU/ml. As a control, a serum collected from a normal woman was tested likewise. The results are shown in Fig. 3, where A is an embodiment of a reference using monoclonal antibody A as the solid phase and No. 6 clone as the labelled antibody, and B is an embodiment of this invention using No. 4 clone as the solid phase and No. 2 clone as the labelled antibody. As is clear from Fig. 3, in case of using monoclonal antibody A, the reaction was significantly inhibited by the presence of HCG, but on the other hand, in case of the system of this invention, HCG did not affect the measurement.

What is claimed is:

1. A method for the measurement of TSH by an immunoassay comprising subjecting a test sample containing TSH as an antigen to an antigen-antibody reaction with an insolubilized antibody as the solid phase and a labelled antibody, separating the labelled antibody bound to the antigen to be determined from the free labelled antibody and measuring the amount of the bound antigen, characterized by

using an insolubilized TSH-β subunit specific monoclonal antibody as the solid phase and a labelled TSH-β subunit specific monoclonal antibody as the labelled antibody, said TSH-β subunit specific monoclonal antibodies recognizing different epitopes.

2. The method according to claim 1, wherein the immunoassay is a one step sandwich method.

3. The method according to claim 1, wherein the immunoassay is an enzyme immunoassay.

4. The method according to claim 3, wherein the labelled antibody is a peroxidase-labelled TSH-β subunit specific monoclonal antibody.

5. The method according to claim 1, wherein the labelled antibody is a radioisotope-labelled TSH-β subunit specific monoclonal antibody.

*1/4*

Fig. 1

Fig. 2-a

Fig. 2-b

0212522

3/4

Fig. 2-c

Concentration of TSH (µU/ml)

90
80
70
30
20
10
0

Solid phase: No. 4 clone
Labelled antibody: No. 2 clone

4        16        64        256

Amount of FSH   (ng/ml)

Fig. 2-d

Concentration of TSH (µU/ml)

100
90
80
70
30
20
10
0

Solid phase: monoclonal antibody A
Labelled antibody: No. 6 clone

4        16        64        256

Amount of FSH   (ng/ml)

Fig. 3 (A)

Solid phase: Monoclonal antibody A
Labelled antibody: No. 6 clone

Fig. 3 (B)

Solid phase: No. 4 clone
Labelled antibody: No. 2 clone